# EUROPÄISCHE PATENTSCHRIFT

(11) **EP 3 064 231 B1**
(45) Veröffentlichungstag und Bekanntmachung des Hinweises auf die Patenterteilung: **07.11.2018**
(21) Anmeldenummer: 15000635.1
(22) Anmeldetag: 05.03.2015
(51) Int. Cl.: A61L 31/04, A61L 31/16, C08L 1/04

(54) **Mischwerkstoff, Stent und Herstellungsverfahren für einen Stent**
Mixing material, stent and method for making a stent
Matière première composite, stent et procédé de fabrication d'un stent

(43) Veröffentlichungstag der Anmeldung: 07.09.2016
(73) Patentinhaber: Rodermann, Jörg, 52064 Aachen (DE)
(72) Erfinder: Rodermann, Jörg, 52064 Aachen (DE)
(74) Vertreter: Tilmann, Max Wilhelm

(56) Entgegenhaltungen:
- EP-A1- 2 208 482
- DE-U1-202011 100 790
- DE-U1-202012 009 561
- US-A- 2 423 707
- US-A1- 2012 177 719
- US-A1- 2012 190 078
- US-A1- 2015 057 685
- BUENO RONALDO R L ET AL: "Evaluation of the efficacy and safety of a stent covered with biosynthetic cellulose in a rabbit iliac artery model.", THE JOURNAL OF INVASIVE CARDIOLOGY AUG 2009, Bd. 21, Nr. 8, August 2009 (2009-08), Seiten 392-396, XP008177226, ISSN: 1557-2501
- DATABASE WPI Week 201517 Thomson Scientific, London, GB; AN 2015-079198 XP002743437, -& CN 104 174 065 A (QINGDAO BOYITE BIOLOGICAL MATERIAL CO LT) 3. Dezember 2014 (2014-12-03)
- DATABASE WPI Week 201377 2013 Thomson Scientific, London, GB; AN 2013-H83695 XP002743438, -& CN 102 924 613 A (UNIV HAINAN) 13. Februar 2013 (2013-02-13)

## Beschreibung

Die Erfindung betrifft einen Mischwerkstoff, einen Stent und ein Herstellungsverfahren für einen Stent.

Ein Stent ist ein medizinisches Implantat, das in Blutgefäßen und Hohlorganen eingesetzt wird, um diese offen zu halten. Häufige Ursachen für eine Stentimplantation sind pathologische Verengungen der Hohlräume der Blutgefäße und Hohlorgane (Lumen) wie z. B. eine Stenose. Da eine Stentimplantation im Vergleich zu einem chirurgischen Eingriff eine schonende Behandlungsmethode ist, gibt es viele Einsatzgebiete. Beispielsweise werden Stents in Koronararterien, Speiseröhren, Atemwegen, Gallengängen und Harnleitern implantiert.

Stents wurden in den letzten Jahren deutlich weiterentwickelt. Bemerkenswerte Innovationen sind beispielsweise medikamentenbeschichtete, bioresorbierbare oder selbstexpandierende Stents. Stents bestehen mittlerweile aus sehr unterschiedlichen Werkstoffen. Dabei kommen Metalle und Metalllegierungen (z. B. aus Tantal, Titan, Chrom, Eisen und Magnesium) sowie Polymere (z. B. Polyl-L-actat) zum Einsatz. Um die Einsatzmöglichkeiten zu erweitern und die Applikation zu verbessern, wird die Form von Stents stetig weiterentwickelt. So sind Stentgeflechte mitunter sehr kompliziert aufgebaut, um beispielsweise eine Längendehnung ohne Querkontraktion oder das Expandieren eines auf einem Ballonkatheter aufgecrimpten Stent zu erlauben. Teilweise verfügen Stents über Endsegmente, die eine deutlich geringere Radialkraft auf das umgebende Gewebe ausüben als der Mittelteil, um das gesunde Gewebe zu schonen oder zu entlasten. Zudem weisen Stents oft Verankerungselemente auf, um sich in das umgebende Gewebe zu verankern und dort einzuwachsen. Mitunter werden Stents mit Ösen und Markern versehen, um beispielsweise bei der Applikation eine Neupositionierung zu ermöglichen. Dies sind einige wenige Beispiele die zeigen sollen, dass die Stentform immer komplexer wird, um sich den Bedürfnissen der Patienten und des medizinischen Fachpersonals anzupassen.

Bekannte und häufig angewandte Verfahren zum Herstellen von Stents wie das Laserschneiden und das Spritzgießen bieten allerdings nur begrenzte Möglichkeiten der Formgebung.

Beim Laserschneiden wird die fertige Form des Stents mittels eines Lasers aus einem rohrförmigen Rohling herausgeschnitten. Dabei können die sehr hohen Lasertemperaturen die mechanischen Eigenschaften des Stents verschlechtern. Zudem ist es mit dem Laserschneiden kaum möglich, Bereiche unterschiedlicher Dicken und Durchmesser herzustellen. Auch kompliziertere Konstruktionselemente wie beispielsweise Hinterschneidungen sind kaum herstellbar. Ferner handelt es sich beim Laserschneiden um ein materialabtragendes, und damit nicht ressourcenschonendes, Fertigungsverfahren.

Beim Spritzgießen wird typischerweise ein plastifizierter Kunststoff unter hohem Druck in eine Form gespritzt. Die Form besteht aus mindestens einer Matrize, die das Negativ der Außenform des Stents bildet. Des Weiteren besteht die Form aus einem Kern, der das Negativ der Innenform des Stents bildet. Im Vergleich zum Laserschneiden bietet das Spritzgießen mehr, wenn auch nicht unbegrenzte, Möglichkeiten der Formgebung. Allerdings handelt es sich dabei um ein sehr starres Fertigungsverfahren, da die Stentform durch die Form (Matrize und Kern) festgelegt ist. Dies kann bei der Serienanfertigung wirtschaftlich sein, erfordert aber bei jeder neuen Stentform die kostenintensive Herstellung neuer Matrizen und das Umrüsten der Fertigungsvorrichtung. Zudem eignet sich das Spritzgießen hauptsächlich für die Verarbeitung von polymeren Werkstoffen. Metallgießverfahren weisen jedoch die gleichen Nachteile wie das Spritzgießverfahrens auf, da hier ebenfalls feste Formen die Stentform bestimmen.

Aus DE 20 2012 009 561 U1 ist ein Nanostent bekannt, der aus mindestens einer, zwei, drei, vier, fünf oder sechs Lagen bioresorbierbaren Polymer und mindestens einer pharmakologisch aktiven Substanz mittels eines Freiformfertigungsverfahrens (3D Drucken) hergestellt wurde. Der Nanostent kann aus einem Gemisch aus mindestens einem bioresorbierbaren Polymer und mindestens einem Terpen, Terpenoid, Memory Polymer, Chitosan, Stärke, Shellac oder Cellulose bestehen. Aus dieser Schrift ist ferner ein Nanostent bekannt, der aus mindestens einem bioresorbierbaren Metall oder einer bioresorbierbaren Metallegierung besteht, welches mit mindestens einer Substanz beschichtet ist, die eine Proliferationshemmung oder Zellaufbau gewährleistet. Weiterhin bevorzugt sind synthetisch hergestellte Seidenprodukte wie zum Beispiel Spinnenseide, Raupenseide oder synthetische Seide gewonnen aus Ziegenmilch sowie ihrer Derivate, Proteine und deren Abkömmlinge hieraus. In einem geeigneten Lösungsmittel kann die mindestens eine, zwei, drei, vier, fünf oder sechsfach aufzubringende Substanz gelöst, emulgiert, suspendiert oder dispergiert werden. Als aufzubringende Substanzen kommen die oben erwähnten pharmakologisch aktiven Substanzen, die Wirkstoffe, die Terpene oder Terpenoide und/oder die oben beschriebenen bioresorbierbaren Polymere oder Seiden Proteine in Frage.

Vor diesem Hintergrund liegt der Erfindung die Aufgabe zugrunde, einen neuen Mischwerkstoff vorzuschlagen, der sich auch für Stents einsetzen lässt, sowie einen Stent aus einem neuen Mischwerkstoff sowie ein Herstellungsverfahren für einen neuen Stent vorzuschlagen.

Die Aufgabe wird durch die Gegenstände der Ansprüche 1, 4, 6, 11 und 12 gelöst. Vorteilhafte Ausführungsformen sind in den Unteransprüchen und der hiernach folgenden Beschreibung angegeben.

Die Erfindung geht von dem Grundgedanken aus, für einen Mischwerkstoff eine Mischung aus oxidierter Cellulose und Seide, bzw. Seidenbestandteilen einzusetzen. Insbesondere bevorzugt ist die oxidierte Cellulose eine oxidierte Nanocellulose. Oxidierte Nanocellulose (US Pat 2423707 (1947) und US Pat 6627749 B1) ist ein biologisch abbaubares Material. Ein Stent aus oxidierter Nanocellulose kann daher vom Körper abgebaut werden. Die Nanocellulose kann mikrofibrilline Nanocellulose, nanokristalline Nanocellulose oder bakterielle Cellulose sein. Je nach dem Oxidierungsgrad der Cellulose kann dabei die Degradationsgeschwindigkeit des Stents variiert werden. Dabei führt oxidierte Cellulose zur Anziehung von Fibroblasten und Endothelzellen, so dass es zu einem schnellen Einwachsen des Stents kommt. Dies hat den Vorteil, dass hierdurch die Gefahr von Stentthrombosen stark verringert wird und die heute übliche 12monatige und kostspielige duale Antikoagulationsbehandlung nach Stentimplantationen stark verringert werden kann. Zusätzlich verringert oxidierte Cellulose die Freisetzung von Matrixmetalloproteinsen (MMP). Diese weichen Kollagenfasern auf und behindern das Überwachsen des Stents mit funktionsfähigen Endothelzellen. Da MMPs ursächlich dadurch auch für die Entstehung von Herzinfarkten durch Plaquerupturen verantwortlich sind, ergibt sich hiermit eine besondere Eignung des aus dem Mischwerkstoff hergestellten Stents zur Behandlung von akuten Infarkten durch Koronarthrombosen.

Der Mischwerkstoff enthält Seide, bzw. Seidenbestandteile, bspw. das Seidenprotein Fibroin. Der Begriff "Seide" bzw. "Seidenbestandteile" umfasst einerseits die natürlich hergestellte Seide. Diese wird typischerweise aus den Kokons eines Seidenspinners wie z. B. Bombyx mori oder Antheraea pernyi (Schmetterlinge) gewonnen. Als Seidenspinner kommen aber auch andere Insekten sowie Spinnen oder sonstige Lebewesen (etwa Muscheln) infrage.

Die natürliche Seide besteht hauptsächlich aus Fibroin. Fibroin ist ein Strukturprotein und weist insbesondere sehr stabile und reißfeste Strukturen und hydrophobe Eigenschaften auf. Sericin ist ein weiterer Bestandteil von Seide. Sericin ist ein Strukturprotein, das insbesondere die Fibroinfasern umgibt und dafür sorgt, dass diese miteinander verkleben. Sericin wirkt somit als Seidenleim und wird bei der Verarbeitung der natürlichen Seide zu Textilprodukten durch das Entbasten entfernt.

Des Weiteren umfasst der Begriff "Seide" bzw. "Seidenbestandteile" alle synthetisch hergestellten, künstlichen oder (chemisch) modifizierten Seiden bzw. Seidenbestandteile.

Das Seidenprotein Fibroin eignet sich hervorragend als Werkstoff für Stents.

Vorzugsweise kann das Seidenprotein Fibroin in Form von Seidenpulver (US Pat 4253212 A 1979; Otoi et al.) verwendet werden.

Der Begriff "Mischung" bedeutet jegliche Kombination der Stoffe, ohne dass durch die Mischung ein neuer Stoff entsteht, also ohne dass zwingend die Notwendigkeit besteht, dass die für die Mischung eingesetzten Stoffe miteinander interagieren, insbesondere ohne dass die Notwendigkeit besteht, dass die für die Mischung eingesetzten Stoffe miteinander reagieren. Der Begriff Mischung schließt aber auch ausdrücklich die Bildung neuer Stoffe ein. Eine "Mischung" kann auch durch eine chemische Reaktion zwischen den für die Mischung eingesetzten Stoffen entstehen. Die Mischung kann ein Gemenge, eine Legierung, ein Komposit, ein Schwamm, ein Schaum sein. Insbesondere kann es sich um homogene Mischung bestehend aus einer Phase oder um heterogene Mischungen mit zwei oder mehr Phasen handeln. Eine "Mischung" kann auch vorliegen, wenn sich die Stoffe nicht mehr in die Ausgangsstoffe auftrennen lassen. Es ist bei der "Mischung" auch möglich, dass die Stoffe ihre ursprünglichen Eigenschaften verlieren, bzw. neue erhalten.

In einer bevorzugten Ausführungsform enthält der Mischwerkstoff zudem noch Cellulase und/oder nicht-oxidierte Cellulose, insbesondere nicht-oxidierte Nanocellulose und/oder Mischungen aus 2 oder mehreren Cellulosesearten. Cellulose ist ein besonders von Pflanzen gebildeter Stoff und Hauptbestandteil der pflanzlichen Zellwände. Cellulose ist daher in großen Mengen vorhanden. In einer besonders bevorzugten Ausführungsform weist der Mischwerkstoff Nanocellulose auf. Nanocellulose kann auf verschiedene Weisen aus Cellulose gewonnen werden: Bei der Herstellung von mikrofibrillierter Cellulose werden Fibrillen beispielsweise in Homogenisatoren durch hohen mechanischen Druck aus der Cellulose isoliert. Nanokristalline Cellulose wird hingegen durch Hydrolyse von Cellulose gewonnen. Die Cellulose befindet sich dabei beispielsweise in Holz oder Holzzellstoff.

Nanocellulose kann auch synthetisch mittels Bakterien hergestellt werden (bakterielle Nanocellulose). Die Bakterien befinden sich in einem Medium, in dem sie Stoffe in Nanocellulose umwandeln. Die Nanocellulose entsteht dabei an der Grenze zwischen dem Medium und der Umgebung, insbesondere Luft. Als Bakterium wird beispielsweise Gluconacetobacter genutzt. Eigenschaften der bakteriellen Nanocellulose (z. B. Zugsfestigkeit und Porosität) können gezielt im Herstellprozess beeinflusst werden. Dies kann etwa durch die Wahl des Mediums oder des Bakteriums geschehen. Einflussgrößen können z. B. auch die Viskosität des Mediums und die Zugabe von Zusatzstoffen, z. B. Nährstoffen für die Bakterien, sein.

Nanocellulose weist hervorragende mechanische Eigenschaften auf. Sie kann beispielsweise eine höhere Festigkeit als Stahl oder Kevlar aufweisen. Gleichzeitig ist Nanocellulose reißfest. Sie eignet sich daher besonders gut als Material für Stents: Stents können in einem Gefäß oder Hohlorgan besonders hohen statischen und dynamischen Belastungen ausgesetzt sein. Insbesondere muss ein Stent hohen Radialkräften standhalten können, um das Lumen offen zu halten. Gleichzeitig muss ein Stent elastisch und flexibel sein, um beispielsweise durch gewundene Körperstellen zur Implantationsstelle geführt oder mittels eines Ballonkatheters an der Verengungsstelle expandiert zu werden.

Nanocellulose weist zudem eine hohe Biokompatibilität auf. Biokompatibel bedeutet, dass ein Material körperverträglich ist und insbesondere keine allergischen oder giftigen Reaktionen auslöst. Es erfolgen keine immunologischen Abwehrreaktionen des Körpers, sodass biokompatible Materialien sich besonders gut für Implantate wie Stents eignen.

Besonders vorteilhaft wirkt sich dies bei Stents aus, die medizinisch bedingt nicht dauerhaft im Körper verbleiben können. Ein zweiter operativer Eingriff zur Entfernung des Stents kann dem Patienten in diesem Fall erspart bleibt.

Stents aus Nanocellulose können auch als medikamentenfreisetzende Stents eingesetzt werden. Diese werden insbesondere zur Prävention von Restenosen benötigt. Bei einer Restenose wird die Implantationsstelle durch die Neubildung von Bindegewebe erneut verengt oder verschlossen. Wirkstoffe wie beispielsweise Paclitaxel oder Sirolimus können die Gewebeneubildung hemmen. Aufgrund der biologischen Abbaubarkeit der oxidierten Nanocellulose können Wirkstoffe zudem über einen längeren Zeitraum sukzessive freigesetzt werden.

Insbesondere bevorzugt wird Baumaterial verwendet, das eine Kombination oder Mischung aus dem Seidenprotein Fibroin und oxidierter Cellulose, insbesondere Nanocellulose, aufweist. Dadurch wird die Flexibilität des Stents im Verhältnis zu allein aus Cellulose hergestellten Stents verbessert.

Der erfindungsgemäße Stent besteht aus einem Mischwerkstoff, der oxidierte Cellulose, insbesondere oxidierte Nanocellulose und/oder Cellulase und/oder nicht-oxidierte Nanocellulose und/oder Mischungen aus 2 oder mehreren Cellulosesearten enthält. In einer bevorzugten Ausführungsform weist der Mischwerkstoff, aus dem der Stent besteht, zudem Seide und/oder Seidenbestandteilen auf.

Das erfindungsgemäße Verfahren zum Herstellen eines Stents sieht vor, dass der Stent mittels eines Freiformfertigungsverfahrens aus einem Baumaterial, das oxidierte Cellulose, insbesondere oxidierte Nanocellulose und/oder Cellulase und/oder nicht-oxidierte Nanocellulose und/oder eine Mischung aus 2 oder mehreren Cellulosesearten und/oder ein nicht-bioresorbierbares Metall und/oder eine nicht-bioresorbierbares Metalllegierung enthält, hergestellt wird.

Das erfindungsgemäße Verfahren sieht im Hinblick auf die Herstellung eines Halbzeugs, aus dem eine Stent hergestellt werden kann vor, dass das Halbzeug mittels eines Freiformfertigungsverfahrens aus einem Baumaterial, das oxidierte Cellulose, insbesondere oxidierte Nanocellulose und/oder Cellulase und/oder nicht-oxidierte Nanocellulose und/oder eine Mischung aus 2 oder mehreren Cellulosesearten und/oder ein nicht-bioresorbierbares Metall und/oder eine nicht-bioresorbierbares Metalllegierung und/oder ein bioresorbierbares Metall und/oder eine bioresorbierbare Metalllegierung und/oder Polylactide enthält, hergestellt wird.

Das Freiformfertigungsverfahren kann mit einer Freiformfertigungsvorrichtung durchgeführt werden. Als Freiformfertigungsvorrichtung ist erfindungsgemäß eine Fertigungsvorrichtung zu verstehen, die in der Lage ist, aus einem oder mehreren Baumaterialien ein Freiformobjekt herzustellen. Freiformobjekt bedeutet, dass das Objekt jede denkbare Form aufweisen kann. Dadurch sind komplexe Formen möglich, die durch andere Fertigungsvorrichtungen nicht gebildet werden können. Insbesondere wird das Freiformobjekt schichtweise aufgebaut. Schichten aus Baumaterial werden dabei beispielsweise durch Verkleben, Verschweißen oder Aushärten miteinander verbunden.

Das erfindungsgemäße Verfahren umfasst sowohl die Verarbeitung eines Baumaterials als auch die Verarbeitung mehrerer Baumaterialien zu einem Freiformobjekt. Der Begriff "Verarbeitung" ist weit auszulegen. Hierzu gehört beispielsweise das Verschmelzen, Aushärten, Verkleben, Positionieren, Transportieren, Bereitstellen und alle Tätigkeiten und Vorgänge, die in einer Verarbeitung oder Fertigung vorkommen. Insbesondere bevorzugt bezieht sich "Verarbeitung" auf alle Tätigkeiten und Vorgänge, bei denen das Baumaterial verändert wird und die zur Veränderung des Baumaterials erforderlich sind.

Freiformfertigungsvorrichtungen sind aus anderen technischen Bereichen wie z. B. der Automobilindustrie und dem Maschinenbau bekannt. Sie werden beispielsweise zur Herstellung von Prototypen (Rapid Prototyping) oder zum Herstellen von Fertigteilen (Rapid Manufacturing) verwendet. Beispiele für im Rahmen der Erfindung einsetzbare Freiformfertigungsverfahren sind 3D-Drucken, selektives Lasersintern, Stereolithographie und Fused Deposition Modeling.

Vorzugsweise erfolgt die Verarbeitung des Baumaterials bzw. der Baumaterialien zu einem Freiformobjekt mittels der Freiformfertigungsvorrichtung computergesteuert. Besonders bevorzugt werden dabei Programme, insbesondere CAD-Programme, verwendet. Daten könne beispielsweise durch eine rechnerunterstütze Konstruktion oder etwa mittels eines Scanverfahrens, z. B. der Computertomographie, erzeugt werden. Die Programmdaten werden vorzugsweise über Dateninterfaces wie die STL-Schnittstelle an die Freiformfertigungsvorrichtung weitergeleitet, sodass das Freiformobjekt gemäß dem Programm gefertigt werden kann.

Als Baumaterial wird erfindungsgemäß ein Material verstanden, das mittels der Freiformfertigungsvorrichtung zumindest zu einem Teil des Freiformobjekts verarbeitet und somit Bestandteil des Freiformobjekts wird. Das Baumaterial kann im unverarbeiteten Zustand (Rohmaterial) jeden Aggregatzustand und jede Form aufweisen. Bei der Stereolithographie kann das Baumaterial etwa als flüssiges Photopolymer in einem Behälter vorliegen, das durch Bestrahlung mittels einer UV-Lichtquelle schichtweise zu einem Freiformobjekt ausgehärtet wird. Beim selektiven Lasersintern wird pulverförmiges Baumaterial verwendet, das üblicherweise auf einem Pulverbett liegt und durch Lasereinstrahlungen an definierten Stellen gesintert wird.

Das erfindungsgemäße Verfahren eignet sich zum Herstellen sämtlicher Stents. Hierzu zählen auch Stentgrafts, die insbesondere einen Stent und eine Gefäßprothese umfassen können, sowie alle ähnlichen Implantate.

Durch die freie Formgebung und die Kombinationsmöglichkeiten von Baumaterialien und Werkstoffen schafft die Erfindung nahezu unbegrenzte Möglichkeiten zur Fertigung von Stents. Stents können entsprechend den Bedürfnissen der Patienten und des medizinischen Fachpersonals ohne Rücksichtnahme auf technische Grenzen bekannter Fertigungsvorrichtungen weiterentwickelt werden. Beispielsweise können Stents eine auf komplexen mathematischen Berechnungen basierende Struktur aufweisen, die eine optimale Stützfunktion ausübt, aber zugleich schonend auf das umgebende Gewebe einwirkt. Gewebeverletzungen, Reizungen oder Restenosen können dadurch verhindert werden. Auch können beispielsweise mithilfe von Computertomographien individuell auf den Patienten angepasste Stentformen problemlos gefertigt werden.

Gleichzeitig bietet die Erfindung ein flexibles und wirtschaftliches Fertigungsverfahren für Stents. Stents unterschiedlicher Formen können in beliebiger Reihenfolge ohne nennenswerten Aufwand, z. B. durch Umrüstung der Fertigungsvorrichtung oder Bestellung neuer Werkzeuge, hergestellt werden. Es ist beispielsweise ausreichend, eine neue Konstruktionsdatei einzulesen oder abzurufen und danach den Fertigungsprozess zu starten.

Als Halbzeug wird ein Erzeugnis eines Herstellungsprozess-Schritts verstanden, das sich in einem oder mehreren nachfolgenden Prozess-Schritten in ein Endprodukt, hier einen Stent, verarbeiten lässt. Ein Halbzeug kann beispielsweise ein Stab oder ein Hohlzylinder sein, der durch Laserschneide-Prozesse in einen Stent verarbeitet wird. Das für das Freiformfertigungsverfahren bei der Herstellung des Halbzeugs verwendete Baumaterial kann auch Polylactide und/oder Polyglycolid und/oder Polycaprolacton und/oder eines der nachfolgenden Polymere: Glycolid/Lactid-Copolymere, Glycolid/Trimethylencarbonat-Copolymere, Poly-L-Iactid, Poly-D-Iactid, Poly-DL-lactid, L-Lactid/DL-Lactid-Copolymere, L-Lactid/D-Lactid-Copolymere, Lactid/Tetramethylenglycolid-Copolymere, Lactid/Trimethylencarbonat-Copolymere, Lactid/δ-Valerolacton-Copolymere, Lactid/ε-Caprolacton-Copolymere, Polydepsipeptide, Glycin-DL-lactid-Copolymer, Polylactid/Ethylenoxid- Copolymere, asymmetrisch 3,6-substituierte Poly-1,4-dioxan-2,5-dione, Poly-β-hydroxybutyrat, Poly-β-hydroxybutyrat/β Hydroxyvalerat- Copolymere, Poly- β-hydroxypropionat, Poly- β-dioxanon, Poly-δ-valerolacton, Poly-ε-caprolacton, Methylmethacrylat-N-Vinylpyrrolidon- Copolymere, Polycarbolactone, Polyesteramide, Polyorthoester, Polyester von Oxalsäure, Polydihydropyrane, Polyalkyl-2-cyanoacrylate, Polyurethane, Polyvinylalkohol, Polypeptide, Poly-β-maleinsäure, Poly-β-alkansäuren, Polyethylenoxid, Chitinpolymere und Copolymere und/oder Mischungen der vorgenannten Polymere.

In einer bevorzugten Ausführungsform wird als Baumaterial für das Halbzeug Zink- und Zinklegierungen, Edelstahl (316L), Cobaltchromium (beispielsweise in Form der folgenden Produkte: Common Name: MP35N, ASTM Material Designation: 35Co-35Ni-20Cr-10Mo, UNS: R30035, ASTM: F562, ISO: 5832-6; Common Name: Phynox, ASTM Material Designation: 40Co-20Cr-16Fe-15Ni- 7Mo-, UNS: R30008, ASTM: F1058, ISO: 5832-7; Common Name: Elgiloy, ASTM Material Designation: 40Co-20Cr-16Fe-15Ni-7Mo-, UNS: R30003, ASTM: F1058, ISO: 5832-7; Common Name: L605, ASTM Material Designation: Co-20Cr-15W-10Ni, UNS: R30605, ASTM: F90, ISO: 5832-5), Magnesium- und/oder eine Magnesiumlegierungen oder Mischungen der vorgenannten Materialien verwendet.

Ferner betrifft die Erfindung einen nach dem erfindungsgemäßen Verfahren hergestellten Stent, bzw. als Zwischenstufe in dem erfindungsgemäßen Verfahren hergestellte Halbzeug. Ein derartiger Stent, bzw. ein aus einem derartigen Halbzeug hergestellter Stent weist gegenüber mittels bekannter Fertigungsvorrichtungen hergestellten Stents diverse Vorteile auf: Er kann beispielsweise eine individuell auf den Patienten zugeschnittene Form und Struktur aufweisen, sodass eine äußerst verträgliche Implantation stattfindet kann. Ein derartiger Stent kann zudem aus einer Vielzahl aus Werkstoffen und Werkstoffkombinationen bestehen, wie kaum ein anderer Stent. Dadurch können je nach Einsatzgebiet und Anwendungszweck verschiedene Eigenschaften wie z. B. Biokompatibilität, Festigkeit und Elastizität bestens genutzt werden. Dabei lässt sich durch Einsatz Cellulose oder Mischungen aus 2 oder mehreren Cellulosesearten, z.b. mit Nanocellulose in dem erfindungsgemäßen Verfahren ein nicht bioabsorbierbaren Stents herstellen. Durch Einsatz von oxidierte Cellulose gemischt mit Seidenfibroin lässt sich mit dem erfindungsgemäßen Verfahren ein bioabsorbierbarer Stent herstellen. Durch Einsatz von Magnesium- oder Magnesiumlegierungen mit oder ohne Beimischung von Seidenfibroin lässt sich mit dem erfindungsgemäßen Verfahren ein bioabsorbierbarer Stents herstellen. Durch Einsatz Zink- oder Zinklegierungen mit oder ohne Beimischung von Seidenfibroin lässt sich mit dem erfindungsgemäßen Verfahren ein bioabsorbierbarer Stent herstellen. Dabei lässt sich durch Edelstahl (316L) oder Cobaltchromium in dem erfindungsgemäßen Verfahren ein nicht bioabsorbierbaren Stents herstellen.

## Patentansprüche

1. Mischwerkstoff, der oxidierte Cellulose gemischt mit Seide und/oder Seidenbestandteilen enthält, wobei die oxidierte Cellulose und die Seide und/oder die Seidenbestandteile durch eine chemische Reaktion gemischt wurden.

2. Mischwerkstoff nach Anspruch 1, **dadurch gekennzeichnet, dass** der Mischwerkstoff Seidenfibroin enthält.

3. Mischwerkstoff nach einem der Ansprüche 1 oder 2, **dadurch gekennzeichnet, dass** der Mischwerkstoff Cellulase und/oder nicht-oxidierte Nanocellulose und/oder mehrere Cellulosesearten enthält.

4. Stent aus einem Mischwerkstoff, gemäss Anspruch 1-3, der oxidierte Cellulose und/oder Cellulase und/oder nicht-oxidierte Nanocellulose und/oder Mischungen aus 2 oder mehreren Cellulosesearten enthält, wobei die oxidierte Cellulose und/oder Cellulase und/oder nicht-oxidierte Nanocellulose und/oder die Mischungen aus 2 oder mehreren Cellulosearten durch eine chemische Reaktion gemischt wurden.

5. Stent aus einem Mischwerkstoff nach einem der Ansprüche 1 bis 3.

6. Verfahren zum Herstellen eines Stents gemäss Anspruch 4 oder 5 **dadurch gekennzeichnet, dass** der Stent mittels Rapid Prototyping, Rapid Manufacturing, 3D-Drucken, selektives Lasersintern, Stereolithografie oder Fused Deposition Modeling aus einem Werkstoff, der oxidierte Cellulose und/oder Cellulase und/oder nicht-oxidierte Nanocellulose und/oder eine Mischung aus 2 oder mehreren Cellulosesearten und/oder ein nicht-bioresorbierbares Metall und/oder eine nicht-bioresorbierbares Metalllegierung enthält, hergestellt wird.

7. Verfahren zum Herstellen eines Stents gemäss Anspruch 6 **dadurch gekennzeichnet, dass** der Stent aus einem Halbzeug hergestellt wird und das Halbzeug mittels Rapid Prototyping, Rapid Manufacturing, 3D-Drucken, selektives Lasersintern, Stereolithografie oder Fused Deposition Modeling aus einem Werkstoff, der oxidierte Cellulose, insbesondere oxidierte Nanocellulose und/oder Cellulase und/oder nicht-oxidierte Nanocellulose und/oder eine Mischung aus 2 oder mehreren Cellulosesearten und/oder ein nicht-bioresorbierbares Metall und/oder eine nicht-bioresorbierbares Metalllegierung und/oder ein bioresorbierbares Metall und/oder eine bioresorbierbare Metalllegierung und/oder Polylactide und/oder Polyglycolid und/oder Polycaprolacton enthält, hergestellt wird.

8. Verfahren nach Anspruch 6 oder 7, **dadurch gekennzeichnet, dass** der Stent oder das Halbzeug nach Durchführung des Rapid Prototyping, Rapid Manufacturing, 3D-Drucken, selektives Lasersintern, Stereolithografie oder Fused Deposition Modeling mit einem Medikament beschichtet wird.

9. Verfahren nach einem der Ansprüche 6 bis 8, **dadurch gekennzeichnet, dass** der Stent oder das Halbzeug nach Durchführung des Rapid Prototyping, Rapid Manufacturing, 3D-Drucken, selektives Lasersintern, Stereolithografie oder Fused Deposition Modeling mit einem absorbierbaren Polymer beschichtet wird.

10. Verfahren nach einem der Ansprüche 6 bis 9, **dadurch gekennzeichnet, dass** der Stent oder das Halbzeug nach Durchführung des Rapid Prototyping, Rapid Manufacturing, 3D-Drucken, selektives Lasersintern, Stereolithografie oder Fused Deposition Modeling mit einem medikamentenfreisetzenden absorbierbaren oder nichtabsorbierbaren Polymer beschichtet wird.

11. Verwendung eines Stents nach einem der Ansprüche 4 oder 5 als Träger zur Herstellung einer künstlichen Aortenklappe zur transkutanen Implantation.

12. Stent oder Halbzeug, hergestellt nach einem Verfahren gemäß der Ansprüche 6 bis 10.

## Claims

1. Mixing material, containing oxidised cellulose mixed with silk and/or silk components, wherein the oxidised cellulose and the silk and/or the silk components have been mixed by a chemical reaction.

2. Mixing material according to claim 1, **characterised in that** the mixing material contains silk fibroin.

3. Mixing material according to any one of claims 1 or 2, **characterised in that** the mixing material contains cellulase and/or non-oxidised nanocellulose and/or more types of cellulose.

4. Stent from a mixing material, according to claims 1 to 3, containing oxidised cellulose and/or cellulase and/or non-oxidised nanocellulose and/or mixtures of 2 or more types of cellulose, wherein the oxidised cellulose and/or cellulase and/or non-oxidised nanocellulose and/or the mixtures of 2 or more types of cellulose have been mixed by a chemical reaction.

5. Stent from a mixing material according to any one of claims 1 to 3.

6. Method of manufacturing a stent according to claim 4 or 5 **characterised in that** the stent is manufactured by means of rapid prototyping, rapid manufacturing, 3D printing, selective laser sintering, stereolithography or fused deposition modelling from a material containing oxidised cellulose and/or cellulase and/or non-oxidised nanocellulose and/or a mixture of 2 or more types of cellulose and/or a non-bioresorbable metal and/or a non- bioresorbable metal alloy.

7. Method of manufacturing a stent according to claim 6 **characterised in that** the stent is manufactured from a semi-finished product and the semi-finished product is manufactured by means of rapid prototyping, rapid manufacturing, 3D printing, selective laser sintering, stereolithography or fused deposition modelling from a material containing oxidised cellulose, in particular oxidised nanocellulose and/or cellulase and/or non-oxidised nanocellulose and/or a mixture of 2 or more types of cellulose and/or a non-bioresorbable metal and/or a non-bioresorbable metal alloy and/or a bioresorbable metal and/or a bioresorbable metal alloy and/or polylactides and/or polyglycolide and/or polycaprolactone.

8. Method according to claim 6 or 7, **characterised in that** the stent or the semi-finished product is coated with a drug after performance of the rapid prototyping, rapid manufacturing, 3D printing, selective laser sintering, stereolithography or fused deposition modelling.

9. Method according to any one of claims 6 to 8, **characterised in that** the stent or the semi-finished product is coated with an absorbable polymer after performance of the rapid prototyping, rapid manufacturing, 3D printing, selective laser sintering, stereolithography or fused deposition modelling.

10. Method according to any one of claims 6 to 9, **characterised in that** the stent or the semi-finished product is coated with a drug-releasing, absorbable or non-absorbable polymer after performance of the rapid prototyping, rapid manufacturing, 3D printing, selective laser sintering, stereolithography or fused deposition modelling.

11. Application of a stent according to any one of claims 4 or 5 as a carrier to produce an artificial aortic valve by transcutaneous implantation.

12. Stent or semi-finished product, manufactured by a method according to claims 6 to 10.

## Revendications

1. Matériau composite qui contient de la cellulose oxydée mélangée à de la soie et/ou à des composants de soie, la cellulose oxydée et la soie et/ou les composants de soie ayant été mélangés par une réaction chimique.

2. Matériau composite selon la revendication 1, **caractérisé en ce que** le matériau composite contient de la fibroïne de soie.

3. Matériau composite selon l'une des revendications 1 ou 2, **caractérisé en ce que** le matériau composite contient de la cellulase et/ou de la nanocellulose non oxydée et/ou plusieurs sortes de cellulose.

4. Stent d'un matériau composite selon les revendications 1-3, qui contient de la cellulose oxydée et/ou de la cellulase et/ou de la nanocellulose non oxydée et/ou des mélanges de 2 ou de plusieurs sortes de cellulose, la cellulose oxydée et/ou la cellulase et/ou la nanocellulose non oxydée et/ou les mélanges de 2 ou de plusieurs sortes de cellulose ayant été mélangés par une réaction chimique.

5. Stent d'un matériau composite selon l'une des revendications 1 à 3.

6. Procédé pour la fabrication d'un stent selon la revendication 4 ou 5, **caractérisé en ce que** le stent est fabriqué à l'aide de prototypage rapide, de fabrication rapide et d'impression 3D, de frittage laser sélectif, de stéréolithographie ou de dépôt de fil fondu d'un matériau qui contient de la cellulose oxydée et/ou de la cellulase et/ou de la nanocellulose non oxydée et/ou un mélange de 2 ou plusieurs sortes de cellulose et/ou un métal non biorésorbable et/ou un alliage métallique non biorésorbable.

7. Procédé pour la fabrication d'un stent selon la revendication 6, **caractérisé en ce que** le stent est fabriqué à partir d'un produit semi-fini et ledit produit semi-fini est fabriqué à l'aide de prototypage rapide, de fabrication rapide et d'impression 3D, de frittage laser sélectif, de stéréolithographie ou de dépôt de fil fondu d'un matériau qui contient de la cellulose oxydée, notamment de la nanocellulose oxydée et/ou de la cellulase et/ou de la nanocellulose non oxydée et/ou un mélange de 2 ou plusieurs sortes de cellulose et/ou un métal non biorésorbable et/ou un alliage métallique non biorésorbable et/ou un métal biorésorbable et/ou un alliage métallique biorésorbable et/ou des polylactides et/ou du polyglycolide et/ou du polycaprolactone.

8. Procédé selon la revendication 6 ou 7, **caractérisé en ce que** le stent ou le produit semi-fini est imprégné de médicament après avoir réalisé le prototypage rapide, la fabrication rapide et l'impression 3D, le frittage laser sélectif, la stéréolithographie ou le dépôt de fil fondu.

9. Procédé selon l'une des revendications 6 à 8, **caractérisé en ce que** le
stent ou le produit semi-fini est revêtu d'un polymère absorbable après
avoir réalisé le prototypage rapide, la fabrication rapide et l'impression 3D, le frittage laser sélectif, la stéréolithographie ou le dépôt de fil fondu.

10. Procédé selon l'une des revendications 6 à 9, **caractérisé en ce que** le stent ou le produit semi-fini est revêtu d'un polymère absorbable ou non absorbable à élution médicamenteuse après avoir réalisé le prototypage rapide, la fabrication rapide et l'impression 3D, le frittage laser sélectif, la stéréolithographie ou le dépôt de fil fondu.

11. Utilisation d'un stent selon la revendication 4 ou 5 comme support pour la fabrication d'une valvule aortique artificielle destinée à une implantation transcutanée.

12. Stent ou produit semi-fini fabriqué selon un procédé selon les revendications 6 à 10.
